(19) Europäisches Patentamt
European Patent Office
Office européen des brevets

(11) Veröffentlichungsnummer: **0 061 180**
**A1**

(12) **EUROPÄISCHE PATENTANMELDUNG**

(21) Anmeldenummer: 82102297.7

(22) Anmeldetag: 19.03.82

(51) Int. Cl.³: **C 07 H 13/04**
//A61K31/70, C07C127/15

(30) Priorität: 20.03.81 DE 3111048

(43) Veröffentlichungstag der Anmeldung:
29.09.82 Patentblatt 82/39

(84) Benannte Vertragsstaaten:
AT BE CH DE FR GB IT LI LU NL SE

(71) Anmelder: Stiftung Deutsches Krebsforschungszentrum
Im Neuenheimer Feld 280
D-6900 Heidelberg 1(DE)

(72) Erfinder: Eisenbrand, Gerhard, Dr.
Banngartenstrasse 19 a
D-6902 Sandhausen(DE)

(72) Erfinder: Tang, Wei-ci, Dr. Res. Inst. Medicine Pharmacy
Lai-wu Street II, No. 2a
Quingdao(CN)

(74) Vertreter: Müller-Boré, Deufel, Schön, Hertel
Patentanwälte
Postfach 86 07 20 Siebertstrasse 4
D-8000 München 86(DE)

(54) **Verfahren zur Herstellung von N-(N'(2-Chloräthyl)-N'-nitroso)-carbamoyl-aminosäure-glucosamiden und -tetraacetylglucosamiden.**

(57) Die Erfindung betrifft ein Verfahren zur Herstellung von N-(N'-(2-Chloräthyl)-N'-nitroso)-carbamoyl-aminosäure-glucosamiden und -tetraacetylglucosamiden, indem man N-(2-Chloräthyl)-N-nitroso-carbamoylazid mit Aminosäuren unter Zugabe von schwachen Alkalien in wäßrigalkoholischer Lösung reagieren läßt und die entstandenen N-(N'-(2-Chloräthyl)-N'-nitro)-carbamoyl-aminosäuren nach Isolierung und gegebenenfalls Reinigung in ein geeignetes organisches Lösungsmittelsystem überführt und unter Zusatz eines an sich bekannten geeigneten basischen Peptidkatalysators mit Tetraacylglucosamin umsetzt oder die Nitrosocarbamoylaminosäure zuerst aktiviert und dann mit Glucosamin unter Zugabe eines tertiären Amins umsetzt. Die Aktivierung erfolgt zweckmäßig mit Chlorameisensäureäthylester unter Zugabe von tertiärem Amin. Es eignen sich alle Aminosäuren.

EP 0 061 180 A1

# Verfahren zur Herstellung von N-(N'(2-Chloräthyl)-N'-nitroso)-carbamoyl-aminosäure-glucosamiden und -tetraacetylglucosamiden

N-(2-Chloräthyl)-N-nitroso-harnstoffe (CNU's), z. B. 1,3-bis-(2-Chloräthyl)-1-nitrosoharnstoff (BCNU), 1-(2-Chloräthyl)-3-cyclohexyl-1-nitrosoharnstoff (CCNU) und 1-(2-Chloräthyl)-3-(4-methyl-cyclohexyl)-1-nitrosoharnstoff (MeCCNU), gehören zu den wichtigen antineoplastischen Chemotherapeutika auch in klinischer Hinsicht. Neben der therapeutischen Wirksamkeit weisen diese Substanzen aber auch eine langdauernde, kumulative Toxizität auf das Knochenmark auf (Wassermann et al., Cancer, 36, 1258-1268, 1975).

Eine Ausnahme machen Chlorozotocin (2-(3-Chloräthyl)-3-nitrosoureido-D-glucopyranose) und Tetraacetylchlorozotocin (Anderson et al, Cancer Res. 35, 761 - 765 (1976)), an Glucose gebundene CNU-Derivate die experimentell gute chemotherapeutische Aktivität mit verminderter Knochenmarkstoxizität verbinden.

Im Gegensatz zu bisher vorliegenden Substanzen wie Chlorozotocin und anderen zuckergebundenen CNU-Derivaten (Hayat et al, Cancer Chemother. Pharmacol., 3, 217 - 221 (1979)) wird im vorliegenden Fall der Zucker nicht direkt am Stickstoff in Position 3 . des Nitrosoharnstoffgerüstes gebunden. Vielmehr wird zunächst ein Aminosäurederivat, nämlich eine N-(N'-(2-Chloräthyl)-N'-nitroso)carbamoylaminosäure hergestellt und diese, ohne daß eine Isolierung im präparativen Maßstab erforderlich ist, was jedoch möglich ist, mit Glucosamin oder Tetraacylglucosamin unter Einwirkung eines geeigneten basischen Peptidaktivators oder eines Diacylamins,

vorzugsweise eines Carbodiimids bei Verwendung von Tetraacylglucosamin und von Triäthylamin bei Verwendung von Glucosamin in der Kälte zur Endverbindung umgesetzt.

Die Reaktionen können wie folgt schematisch dargestellt werden:

Umsetzung mit Glucosamin

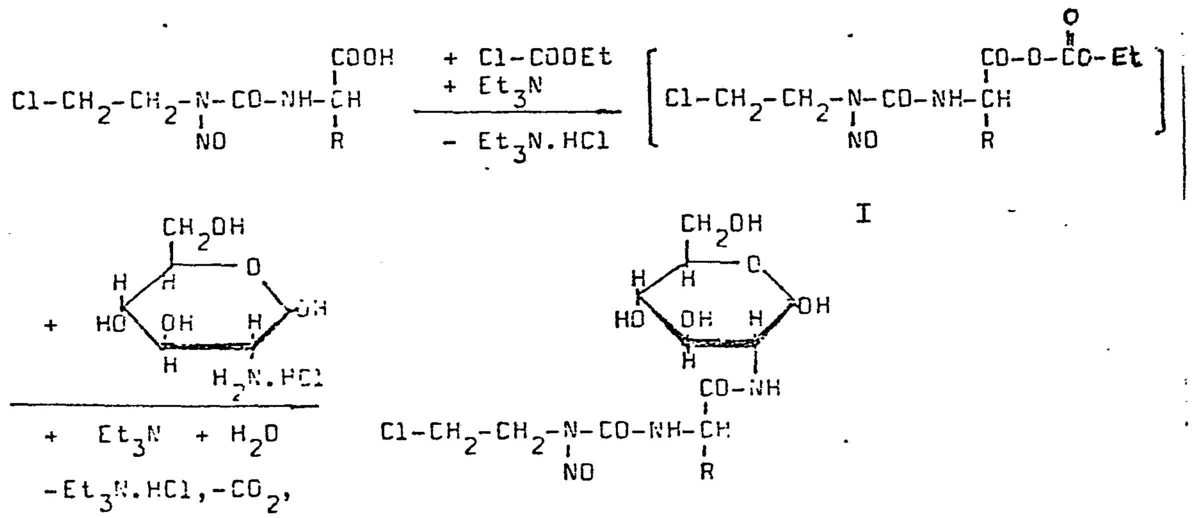


I

$R = CH_3, -CH(CH_3)_2, -CH_2-C_6H_5$

II

Bei der Verwendung des nicht acylierten Glucosamins wird die Nitrosocarbamoylaminosäure in einem organischen Lösungsmittelgemisch, z. B. Dioxan/Tetrahydrofuran 1:1 mit Chlorameisensäureäthylester unter Zugabe von tert. Amin zunächst aktiviert, wobei vermutlich das Zwischenprodukt I gebildet wird; anschließend wird mit Glucosamin unter Zugabe von tertiärem Amin, insbesondere Triäthylamin in der Kälte umgesetzt.

Bei Verwendung von Tetraacylglucosamin kann wie folgt gearbeitet werden:

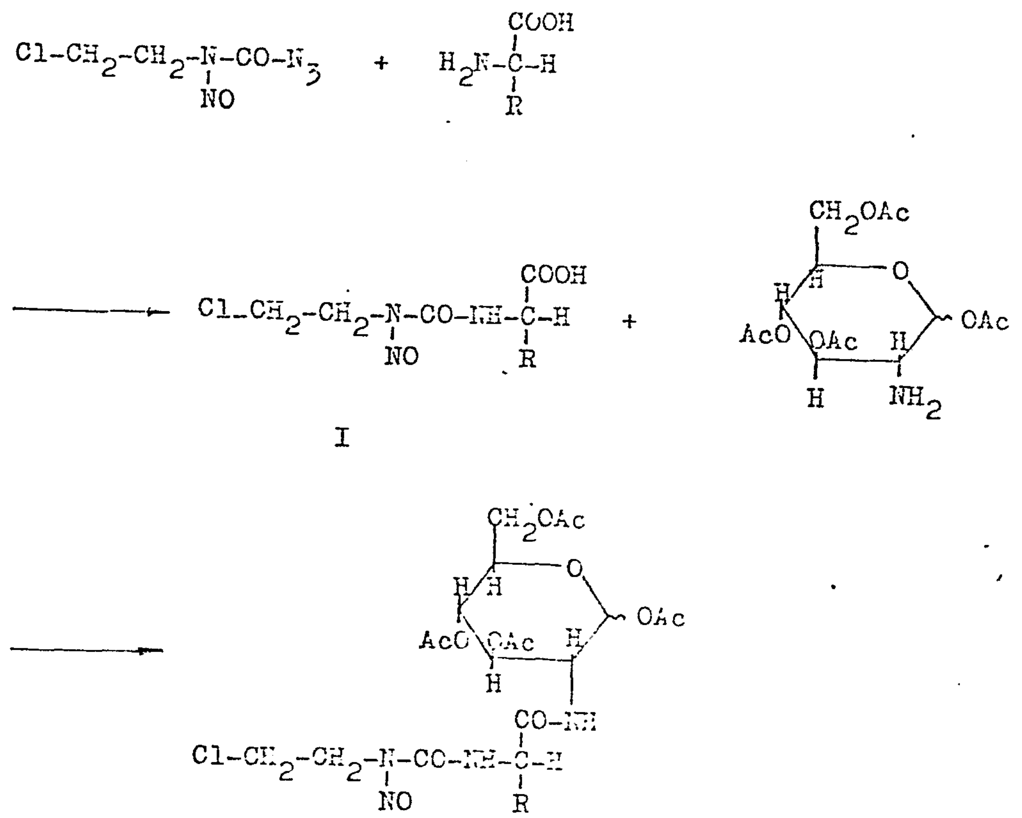

I

II

Die folgenden Beispiele erläutern die Erfindung.

Beispiel 1

Herstellung von N-(N'-(2-Chloräthyl)-N'-nitroso)-carbamoylaminosäuren am Beispiel von N-(N'-(2-Chloräthyl)-N'-nitroso)-carbamoyl-L-alanin

Die Aminosäure (10 mM) wird mit der äquivalenten Menge Natriumbicarbonat in möglichst wenig Wasser gelöst. Diese wäßrige Lösung wird unter Rühren in einer isopropyl-alkoholischen Lösung von N-(2-Chloräthyl)-N-nitroso-carbamoylazid (10 mM) bei 0°C, zugetropft, danach noch 4 Stunden weitergerührt und anschließend über Nacht stehengelassen. Das Reaktionsgemisch wird mit Weinsäure (10 mM) angesäuert und mit Ameisensäure-äthylester extrahiert. Durch Ausschütteln der organischen Phase mit wäßriger Natriumbicarbonatlösung wird das Reaktionsprodukt von Verunreinigungen abgetrennt; nach Ansäuern wird es mit Äther rückextrahiert. Die ätherische Lösung wird getrocknet ($Na_2SO_4$) und das Lösungsmittel abrotiert. Das Rohprodukt kann säulenchromatographisch rein dargestellt werden (Kieselgel, Elution mit Methanol/Chloroform 1:10). Aus der Hauptfraktion wird das reine Produkt nach Entfernen von Lösungsmittel in Vakuum durch Umkristallisieren aus Dichlormethan/n-Pentan erhalten. Hellgelbe Kristalle, Ausbeute 35 %, $F_p$ = 60 - 62°C (Zers.); Elementaranalyse: Ber.: C 32,23; H 4,51; N 18,79; Gef.: C 32,21; H 4,49; N 18,90.

Das NMR-Spektrum (in $CDCl_3$ mit TMS als internen Standard) weist folgende charakteristische Peaks auf: $\delta$ = 1,60 ppm (d, $-CH_3$); $\delta$ = 3,60 ppm (t, $Cl-CH_2-$); $\delta$ = 4,15 ppm (t, $-CH_2-N-NO$); $\delta$ = 4,65 ppm (m, -CH-; $\delta$ = 8,05 ppm (d, -NH-); $\delta$ = 10,65 ppm (s, -COOH).

Die Darstellung der entsprechenden Derivate von L-Valin, L-Phenylalanin, und L-Threonin erfolgte analog[1], jedoch wurde bei letzteren auf eine säulenchromatographische Reinigung verzichtet, d. h. die Verbindungen wurden direkt mit Tetraacetylglucosamin zu den Endprodukten umgesetzt.

Beispiel 2

Herstellung von N-(N'(2-Chloräthyl)-N'-nitroso)-carbamoyl-L-alanin-tetraacetylglucosamin (II, R = $CH_3$).

N-(N'-(2-Chloräthyl)-N'-nitroso)-carbamoyl-L-alanin (1 mM) und Tetraacetylglucosamin (1 mM) werden zusammen in 10 ml Dichlormethan gelöst. Unter Eiskühlung und ständigem Rühren wird eine Lösung von DCC (1,1 mM) in 10 ml Dichlormethan zugetropft. Nach einigen Minuten scheidet sich der dabei gebildete N,N-Dicyclohexylharnstoff aus. Nach einer Stunde ist die Reaktion beendet. Der Niederschlag wird abfiltriert und das Filtrat zur Trockne abrotiert. Das Rohprodukt wird durch Säulenchromatographie gereinigt (Kieselgel, Elution mit Ethylformiat/Dichlormethan (1:1)). Aus der Hauptfraktion wird nach Einengen des Elutionsmittels durch Zugabe von Äther die Reinsubstanz ausgefällt. Ausbeute 75 %; $F_p$ = 122°C (Zersetzung); Elementaranalyse: Ber.: C 42,08; H 5,47; N 9,81; Gef.: C 42,22; H 5,65; N 9,79. NMR-spektroskopische Untersuchung ergab charakteristische Peaks bei: $\delta$=3,50 ppm (t,

---

1) Die Umsetzung mit dem Nitrosocarbamoylazid kann dabei auch bei Raumtemperatur vorgenommen werden.

Cl-$CH_2$-); $\delta$= 4,20 ppm (t, -$CH_2$-N-NO), $\delta$= 6,55 ppm (d, -NH-); $\delta$= 7,35 ppm (d, -NH-).

Beispiel 3

Herstellung von N-(N'-(2-Chloräthyl)-N'-nitroso)-carbamoyl-L-valin-tetraacetylglucosamid (II, R= -$CH(CH_3)_2$).

N-(N'-(2-Chloräthyl)-N'-nitroso)-carbamoyl-L-valin (1 mM) und Tetraacetylglucosamin (1 mM) werden in 10 ml Dichlormethan unter Zugabe von DCC (1,1 mM) bei 0°C umgesetzt. Das Reaktionsgemisch wird nach einer Stunde filtriert und das Filtrat im Vakuum zur Trockne abrotiert. Das Rohprodukt wird säulenchromatographisch gereinigt (Kieselgel, Elution mit Diethylether/Dichlormethan 1:3) und die Reinsubstanz aus Dichlormethan/n-Pentan umkristallisiert. Ausbeute 65 %; $F_p$ = 156°C (Zers.); Elementaranalyse: Ber.: C 45,48; H 5,73; N 9,64; Gef.: C 45,50; H 5,78; N 9,58. Die NMR-spektroskopische Untersuchung ergab charakteristische Peaks bei: $\delta$=3,50 ppm (t, Cl-$CH_2$-); $\delta$= 4,20 ppm (-$CH_2$-N-NO); $\delta$= 6,65 ppm (-NH-); $\delta$ = 7,30 ppm (-NH-).

Beispiel 4

Herstellung von N-(N'-(2-Chloräthyl)-N'-nitroso)-carbamoyl-L-phenylalanin-tetraacetylglucosamid (II, R=-$CH_2$-$C_6H_5$).

Man löst N-(N'-(2-Chloräthyl)-N'-nitroso)-carbamoyl-L-phenylalanin (1 mM) und Tetraacetylglucosamin (1 mM) zusammen in 10 ml Dichlormethan und gibt unter ständigem Rühren und Eiskühlung eine Lösung von DCC (1,1 mM) in 10 ml Dichlormethan dazu. Nach einer Stunde wird

das Reaktionsgemisch filtriert und das Filtrat im Vakuum eingeengt. Das Rohprodukt wird säulenchromatographisch gereinigt (Kieselgel, Elution mit Diethylether/Dichlormethan (1:3)) und die Reinsubstanz nach Abrotieren des Elutionsmittels in wenig Dichlormethan aufgenommen. Nach Zugabe des fünffachen Volumens an Diethylether kristallisiert das Produkt im Kühlschrank in Form von gelben Nadeln. Ausbeute 60 bis 65 %; $F_p$ = 148 bis 149°C (Zers.); Elementaranalyse: Ber.: C 49,65; H 5,29; N 8,91; Gef.: C 49,82; H 5,32; N 8,69. Das NMR-Spektrum zeigt die charakteristischen Peaks von: $\delta$ = 3,45 ppm (t, $Cl-CH_2-$); $\delta$ = 4,20 ppm (t, $-CH_2-N-NO$); $\delta$ = 6,40 ppm (d, -NH-); $\delta$ = 7,10 ppm (d, -NH-); $\delta$ = 7,20 ppm (m, phenyl-).

Beispiel 5

Herstellung von N-(N'-(2-Chloräthyl)-N'-nitroso)-carbamoyl-L-threonin-tetraacetylglucosamid (II, R= $-CH(OH)-CH_3$)

N-(N'-(2-Chloräthyl)-N'-nitroso)-carbamoyl-L-threonin (1 mM) und Tetraacetylglucosamin (1 mM) werden in 10 ml Dichlormethan unter Zusatz von DCC (1,1 mM) bei 0°C umgesetzt.

Nach einer Stunde wird das Reaktionsgemisch filtriert und im Vakuum eingeengt. Das Rohprodukt wird säulenchromatographisch gereinigt (Kieselgel, Elution mit Ethylformiat/Dichlormethan (1:1) und aus Dichlormethan/n-Pentan umkristallisiert. Gelbliche Prismen, Ausbeute 60 %, $F_p$ = 140°C (Zers., sintert bei 87°C). Elementaranalyse: Ber.: C 43,27; H 5,36; N 9,61; Gef.: C 43,18; H 5,42; N 9,40. Die NMR-spektroskopische Untersuchung

ergab charakteristische Peaks von: $\delta$ 3,50 ppm (t, $Cl-CH_2-$); $\delta$ = 4,20 ppm (t, $-CH_2-N-NO$); $\delta$ = 6,9 ppm (d, -NH-); $\delta$ = 7,7 ppm (d, -NH-).

## Beispiel 6

Herstellung von N-(N'-(2-Chlorethyl)-N'-nitroso)-carbamoyl-L-alaninglucosamid

10 mM N-(N'-(2-Chlorethyl)-N'-nitroso)-carbamoyl-L-alanin wird in 100 ml Dioxan/THF (1:1) gelöst. Diese Lösung wird unter ständigem Rühren bei -5°C mit 10 mM (1,4 ml) Triäthylamin und 10 mM (1 ml) Chlorameisensäureäthylester versetzt. Nach 10 Minuten wird das augeschiedene Triäthylaminhydrochlorid rasch abfiltriert. Zu dem Filtrat gibt man eine eiskalte Lösung von 10 mM (2,16 g) D-Glucosaminhydrochlorid in 10 ml Wasser, die 10 mM (1,4 ml) Triäthylamin enthält, hinzu. Die klare Reaktionslösung wird dann 2 Stunden bei Zimmertemperatur weiter gerührt und über Nacht stehengelassen. Das Lösungsmittel wird anschließend in Vakuum bei 40°C verjagt. Der Rückstand wird mit THF aufgenommen und filtriert. Die THF-Lösung wird mit wasserfreiem Natriumsulfat getrocknet und in Vakuum zur Trockne eingeengt. Nach Waschen mit 3 mal 50 ml Äther wird der Rückstand auf einen Filter gesammelt.
Ausbeute: ca. 70 % (Rohprodukt).

## Beispiele 7 und 8

N-(N'-(2-Chloräthyl)-N'-nitroso)-carbamoyl-L-phenylalaninglucosamid und N-(N'-(2-Chloräthyl)-N'-nitroso)-carbamoyl-L-valinglucosamid wurden analog Beispiel 6 und mit etwa gleichen Ausbeuten synthetisiert.

Patentansprüche

1. Verfahren zur Herstellung von N-(N'(2-Chloräthyl)-N'-nitroso)-carbamoyl-aminosäure-glucosamiden und -tetraacetyl-glucosamiden; dadurch gekennzeichnet, daß man N-(2-Chloräthyl)-N-nitroso-carbamoylazid mit Aminosäuren unter Zugabe von schwachen Alkalien in wäßrig-alkoholischer Lösung reagieren läßt und die entstandenen N-(N'-(2-Chloräthyl)-N'-nitroso)-carbamoyl-aminosäuren nach Isolierung und gegebenenfalls Reinigung in ein geeignetes organisches Lösungsmittelsystem überführt und unter Zusatz eines an sich bekannten geeigneten basischen Peptidkatalysators oder eines tertiären Amins mit Glucosamin oder Tetracylglucosamin umsetzt.

2. Verfahren nach Anspruch 1, dadurch gekennzeichnet, daß die Umsetzung mit Tetraacylglucosamin unter Einwirken eines Carbodiimids bei tiefer Temperatur, insbesondere etwa 0°C, erfolgt.

3. Verfahren nach Anspruch 1, dadurch gekennzeichnet, daß zur Umsetzung mit Glucosamin die Carbamoylaminosäure in einem geeigneten organischen Lösungsmittel mit Chlorameisensäureäthylester unter Zugabe von tertiärem Amin zunächst aktiviert und dann mit Glucosamin unter erneuter Zugabe von tertiärem Amin umgesetzt wird.

4. Verfahren nach Anspruch 3, dadurch gekennzeichnet, daß die Aktivierung in einem Lösungsmittelgemisch, insbesondere Dioxan/Tetrahydrofuran 1:1 erfolgt.

5. Verfahren nach Anspruch 4, dadurch gekennzeichnet, daß als tertiäres Amin Triäthylamin verwendet wird.

6. Verfahren nach Anspruch 1, dadurch gekennzeichnet, daß die Umsetzung des Carbamoylazids mit Aminosäure unter Zugabe von Natriumbicarbonat in wäßrig-alkoholischer Lösung bei Zimmertemperatur erfolgt.

7. Verfahren nach Anspruch 1, dadurch gekennzeichnet, daß die Umsetzung mit Tetracylglucosamin in Dichlormethan erfolgt.

8. Verfahren nach Anspruch 1, dadurch gekennzeichnet, daß als Tetraacylglucosamin Tetraacetylglucosamin eingesetzt wird.

9. Verfahren nach einem der vorhergehenden Ansprüche, dadurch gekennzeichnet, daß natürlich vorkommende Aminosäuren oder $\delta$-Aminobuttersäure eingesetzt werden.

Europäisches Patentamt

**EUROPÄISCHER RECHERCHENBERICHT**

**0061180**

Nummer der Anmeldung

EP 82 10 2297

| Kategorie | EINSCHLÄGIGE DOKUMENTE — Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | CHEMICAL ABSTRACTS, Band 91, Nr. 3, 16. Juli 1979, Seite 34, Nr. 13589c, Columbus, Ohio, USA I.S. SOKOLOVA et al.: "Different sensitivity of DNA synthesis in normal and tumor cells to the effect of some N-alkyl-N-nitrosoureas" & DOKL. AKAD. NAUK SSSR 1979, 245(1), 260-264 * Zusammenfassung * --- | 1 | C 07 H 13/04 // A 61 K 31/70 C 07 C 127/15 |
| P,Y | GB-A-2 071 089 (KOWA) * Seiten 11,12 * & DE - A - 3 107 514 --- | 1 | |
| P,Y | CHEMICAL ABSTRACTS, Band 96, Nr. 9, 1. März 1982, Seite 654, Nr. 69391c, Columbus, OHio, USA W.C. TANG et al.: "Synthesis of potentially antineoplastic derivatives of N-(N-(2-chloroethyl)-N-nitrosocarbamoyl)amino acids" & ARCH. PHARM. (WEINHEIM, GER.) 1981, 314(11), 910-917 * Zusammenfassung * --- | 1 | RECHERCHIERTE SACHGEBIETE (Int. Cl 3) C 07 H 13/00 |
| P,Y | CHEMICAL ABSTRACTS, Band 96, Nr. 1, 4. Januar 1982, Seite 641, Nr. 6996h, Columbus, Ohio, USA T. SUAMI et al.: "Synthesis and evaluation of new nitrosoureas congeners" & INSERM SYMP. 1981, 19(NITROSOUREAS CANCER TREAT.), 97-103 * Zusammenfassung * ----- | 1 | |

Der vorliegende Recherchenbericht wurde für alle Patentansprüche erstellt.

| Recherchenort | Abschlußdatum der Recherche | Prüfer |
|---|---|---|
| DEN HAAG | 16-06-1982 | VERHULST W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument